# EUROPÄISCHE PATENTSCHRIFT

(11) **EP 0 832 876 B1**
(45) Veröffentlichungstag und Bekanntmachung des Hinweises auf die Patenterteilung: **31.10.2001**
(21) Anmeldenummer: 97116725.9
(22) Anmeldetag: 25.09.1997
(51) Int. Cl.: C07C 251/60, C07C 49/84, C07C 251/48, C07C 327/44, A01N 37/52

(54) **Difluorochloromethoxybenzolderivate und ihre Verwendung als Schädlingsbekämpfungsmittel**
Difluorochloromethoxybenzene derivatives and their use as pesticides
Dérivés de difluorochlorométhoxybenzènes et leur utilisation comme pesticides

(30) Priorität: 27.09.1996 CH 237596; 01.11.1996 CH 270896
(43) Veröffentlichungstag der Anmeldung: 01.04.1998
(73) Patentinhaber: Bayer Aktiengesellschaft, 51368 Leverkusen (DE)
(72) Erfinder: Ziegler, Hugo, 4108 Witterswil (CH); Zurflüh, René, 4052 Basel (CH)

(56) Entgegenhaltungen:
- EP-A- 0 460 575
- WO-A-94/26700
- WO-A-95/20569

## Beschreibung

Die Erfindung betrifft neue pestizid wirksame Verbindungen der Formel I, 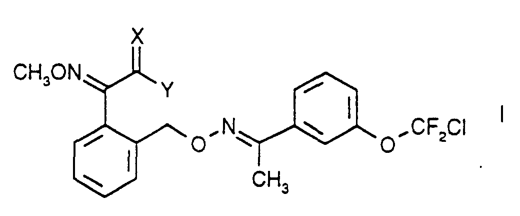 worin bedeuten:
X O und
Y NHCH₃.

Die Verbindungen der Formel I und ihre fungizide Wirkung sind generisch, nicht aber als Einzelverbindungen beschrieben in EP-A-460,575, EP-A-463,488 und WO 94/26700.

Es wurde gefunden, dass sich die erfindungsgemässen Verbindungen durch ausserordentlich gute fungizide Wirkung, insbesondere im Getreide, auszeichnen.

Die Verbindungen der Formel I können gemäss Reaktionsschema nach bekannten Verfahren wie folgt hergestellt werden:

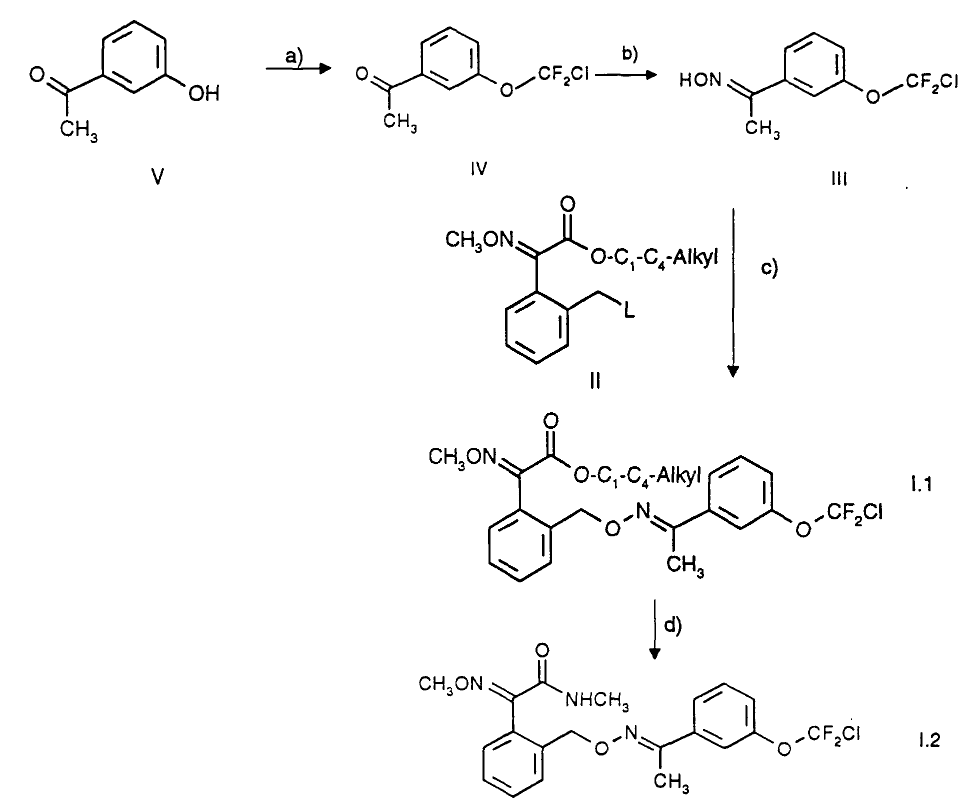

Umsetzung mit
a) Phosgen oder Diphosgen oder CCl₄ in HF;
b) Hydroxylamin oder dessen Salz;
c) Verbindung der Formel II, worin L eine Abgangsgruppe, bevorzugt ein Halogenid, insbesondere Chlorid und Bromid darstellt, unter basischen Bedingungen;
d)Methylamin;

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethan; Ether, wie Diethylether, tert.-Butylmethylether, Tetrahydrofuran oder Dioxan; Ketone, wie Aceton oder Methylethylketon; Alkohole, wie Methanol, Ethanol, Propanol, Butanol, Ethylenglycol oder Glycerin; Ester, wie Essigsäureethylester oder Essigsäurebutylester; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid. Auch im Überschuss eingesetzte Basen, wie Triethylamin, Pyridin, N-Methylmorpholin oder N,N-Diäthylanilin, können als Lösungs- oder Verdünnungsmittel dienen.
Die Umsetzung kann auch unter Phasentransferkatalyse in einem organischen Lösungsmittel, z.B. Methylenchlorid oder Toluol, in Gegenwart einer wässrigen basischen Lösung, z.B. Natriumhydroxid-Lösung, sowie eines Phasentransferkatalysators, z.B. Tetrabutylammoniumhydrogensulfat, erfolgen.

Die neuen Zwischenprodukte der Formeln IV und III sind ebenfalls Gegenstand der Erfindung.

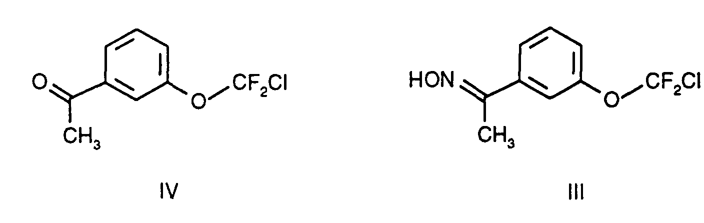

Die Verbindungen der Formel I lassen sich auf dem Agrarsektor und verwandten Gebieten " präventiv und/oder kurativ als Wirkstoffe bei der Bekämpfung von Pflanzenkrankheiten einsetzen. Die erfindungsgemässen Wirkstoffe der Formel I zeichnen sich durch gute Wirkung auch bei niedrigen Anwendungskonzentrationen, durch gute Pflanzenverträglichkeit und Umweltfreundlichkeit aus. Sie besitzen sehr vorteilhafte, insbesondere systemische, Eigenschaften und können zum Schutz von zahlreichen Kulturpflanzen eingesetzt werden. Mit den Wirkstoffen der Formel I können an Pflanzen oder Pflanzenteilen (Früchten, Blüten, Laubwerk, Stengeln, Knollen, Wurzeln) von unterschiedlichen Nutzkulturen die auftretenden Schädlinge eingedämmt oder vemichtet werden, wobei auch später zuwachsende Pflanzenteile, z. B. von phytopathogenen Mikroorganismen, verschont bleiben.

Die Verbindungen I können ferner als Beizmittel zur Behandlung von Saatgut (Früchten, Knollen, Körnern) und Pflanzenstecklingen zum Schutz vor Pilzinfektionen sowie gegen im Erdboden auftretende phytopathogene Pilze eingesetzt werden.

Die Verbindungen I sind z. B. gegen die den folgenden Klassen angehörenden phytopathogenen Pilze wirksam: Fungi imperfecti (z. B. Botrytis, Pyricularia, Helminthosporium, Fusarium, Septoria, Cercospora und Alternaria); Basidiomyceten (z. B. Rhizoctonia, Hemileia, Puccinia); Ascomyceten (z. B. Venturia und Erysiphe, Podosphaera, Monilinia, Uncinula) und Oomyceten (z. B. Phytophthora, Pythium, Plasmopara).

Als Zielkulturen für den pflanzenschützenden Einsatz gelten im Rahmen der Erfindung beispielsweise folgende Pflanzenarten: Getreide (Weizen, Gerste, Roggen, Hafer, Reis, Mais, Sorghum und verwandte Spezies); Rüben (Zucker- und Futterrüben); Kern-, Stein und Beerenobst (Aepfel, Bimen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Campfer) und Pflanzen wie Tabak, Nüsse, Kaffee, Eierfrüchte, Zuckerrohr, Tee, Pfeffer, Reben, Hopfen, Bananen- und Naturkautschukgewächse sowie Zierpflanzen.

Die erfindung betrifft auch ein Mittel zum Schutz von Pflanzen gegen Pilzbefall und ist dadurch gekennzeichnet, dass es als Wirkstoff eine Verbindung der Formel I in freier Form oder in agrochemisch verwendberer Salzform zusammen mit einem Trägermaterial enthält.

Wirkstoffe I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können z. B. Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Die Wirkstoffe der Formel I können mit anderen Fungiziden gemischt werden, wodurch zum Teil unerwartete synergistische Wirkungen entstehen.
Besonders bevorzugte Mischungspartner sind
Azole, wie Propiconazol, Difenoconazol, Cyproconazol, Epoxiconazol, Tebuconazol, Tetraconazol, Fenbuconazol, Metconazol, Bromuconazol;
ferner Fenpropidin, Fenpropimorph, Cyprodinil, Pyrimethanil, Benzo-1,2,3-thiadiazol-7-carbonthiosäure S-methylester;
Strobilurine, wie Azoxystrobin und Kresoxim-methyl.

Geeignete Träger und Zusätze können fest oder flüssig sein und sind in der Formulierungstechnik zweckdienliche Stoffe, z. B. natürliche oder regenerierte mineralische Stoffe, Lösungs-, Dispergier-, Netz-, Haft-, Verdickungs-, Binde- oder Düngemittel.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Bei Wasserreiskulturen kann man solche Granulate dem überfluteten Reisfeld zudosieren. Die Verbindungen I können aber auch zur Saatgutbehandlung auf Samenkörner aufgebracht werden (Coating), indem man die Kömer bzw. Knollen entweder in einer flüssigen Zubereitung des Wirkstoffs tränkt oder sie mit einer festen Zubereitung beschichtet.

Die Verbindungen I werden dabei in unveränderter Form oder vorzugsweise zusammen mit den in der Formulierungstechnik üblichen Hilfsmitteln eingesetzt. Dafür werden sie zweckmässigerweise z. B. zu Emulsionskonzentraten, streichfähigen Pasten, direkt versprühbaren oder verdünnbaren Lösungen, verdünnten Emulsionen, Spritzpulvem, löslichen Pulvern, Stäubemitteln oder Granulaten, z. B. durch Verkapselung in, z. B. polymeren, Stoffen, in bekannter Weise verarbeitet. Die Anwendungsverfahren wie Versprühen, Vernebeln, Verstäuben, Verstreuen, Bestreichen oder Giessen werden ebenso wie die Art der Mittel den angestrebten Zielen und den gegebenen Verhältnissen entsprechend gewählt.

Günstige Aufwandmengen liegen im allgemeinen bei 1 g bis 2 kg Aktivsubstanz (AS) je Hektar (ha), bevorzugt bei 10 g bis 1 kg AS/ha, insbesondere bei 20 g bis 600 g AS/ha. Bei der Verwendung als Saatbeizmittel werden mit Vorteil Dosierungen von 10 mg bis 1 g Aktivsubstanz pro kg Saatgut verwendet.

Die Formulierungen, d. h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen des Wirkstoffs mit Streckmitteln, wie Lösungsmitteln, festen Trägerstoffen und gegebenenfalls oberflächenaktiven Verbindungen (Tensiden).

Als Lösungsmittel kommen in Frage: Aromatische Kohlenwasserstoffe, bevorzugt die Fraktionen C₈ bis C₁₂, wie Xylolgemische oder substituierte Naphthaline, Phthalsäureester, wie Dibutyl- oder Dioctylphthalat, aliphatische Kohlenwasserstoffe, wie Cyclohexan oder Paraffine, Alkohole und Glycole sowie deren Ether und Ester, wie Ethanol, Ethylenglycol, Ethylenglycolmonomethyl- oder -ethylether, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, und Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekömte, adsorptive Granulatträger kommen poröse Typen, wie Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie Dolomit oder zerkleinerte Pflanzenrückstände, verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Beispiele nichtionischer Tenside seien Nonylphenolpolyethoxyethanole, Ricinusölpolyglycolether, Polypropylen-/Polyethylen-oxidaddukte, Tributylphenoxypoly ethylenethanol, Polyethylenglycol und Octylphenoxypolyethoxyethanol erwähnt.
Ferner kommen auch Fettsäureester von Polyoxyethylensorbitan, wie das Polyoxyethylensorbitan-trioleat, in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 Gewichtsprozent, insbesondere 0,1 bis 95 Gewichtsprozent, Wirkstoff der Formel I, 99,9 bis 1 Gewichtsprozent, insbesondere 99,8 bis 5 Gewichtsprozent, eines festen oder flüssigen Zusatzstoffes und 0 bis 25 Gewichtsprozent, insbesondere 0,1 bis 25 Gewichtsprozent, eines Tensids.

Während als Handelsware eher konzentrierte Mittel bevorzugt werden, verwendet der Endverbraucher in der Regel verdünnte Mittel.

Die Mittel können auch weitere Zusätze, wie Stabilisatoren, Entschäumer, Viskositätsregulatoren, Bindemittel oder Haftmittel, sowie Dünger oder andere Wirkstoffe zur Erzielung spezieller Effekte enthalten.

### 1. Herstellungsbeispiele

Temperaturen in °Celsius. Abkürzungen: RT = Raumtemperatur.

### Beispiel H-1:

### Herstellung von 1-(3-(Chloro-difluoro-methoxy)-phenyl)-ethanon (IV)

25 g 3-Hydroxyacetophenon werden im Autoklaven vorgelegt. Nach Abkühlen mittels Trockeneis-Bad werden 94 g HF aufgepresst. Anschliessend werden bei RT 75 g Diphosgen aufgepresst. Nach 6 Tagen wird HF abgesaugt und der Rückstand mit Ethylacetat in Eiswasser eingespült. Das Gemisch wird mit NH₃- Lösung neutral gestellt. Die organ. Phase wird mit ges. NaCI- Lösung zweimal gewaschen und die Waschwasser werden mit Ethylacetat nachextrahiert. Die vereinigten organ. Phasen werden über Natriumsulfat getrochnet. Filtrieren und Eindampfen am Rotavap ergibt ein rotgelbes Oel. Nach Chromatographie an Kieselgel (zur Entfernung des Eduktes) mit Hexan-Diaethylaether 1:1 werden 23,5 g gewünschtes Produktes als gelbes Oel erhalten.
Sdp. 105°C/ 13,33 mbar [=10 mmHg].

### Beispiel H-2:

### Herstellung von 1-(3-(Chloro-difluoro-methoxy)-phenyl)-ethanon oxim (III)

Ein Gemisch aus 3,3 g 1-(3-Chloro-difluoro-methoxy)-phenyl)-ethanon, 1,4 g Hydroxylamin-Hyrochlorid und 10 ml Pyridin wird während 2 Stunden bei 50 ° C gerührt. Dann wird auf Eiswasser gegossen, mit Diethylether extrahiert, am Rotavap eingeengt und noch vorhandenes Pyridin mittels Toluol azeotrop entfemt. Trocknen unter reduziertem Druck bei 60° C ergibt 3,75 g gewünschtes Oxim als Oel.

### Beispiel H-3:

### Herstellung von 2-(1-(3-(Chloro-difluoro-methoxy)-phenyl)-ethylideneaminoxymethyl)-phenyl)-methoximino-essigsäure methyl ester (I.1)

0, 2 g einer 60%igen Natriumhydrid-Dispersion in Mineralöl werden mit Hexan gewaschen und mit 5 ml N,N-Dimethylformamid versetzt. Zu dieser Suspension gibt man 1,43 g 2-(-Brommethylphenyl)glyoxylsaeure-methylester-O-methyloxim und 1,18 g 1-(3-Chlorodifluoro-methoxy)-phenyl)-ethanon oxim. Das Raktionsgemisch wird auf 50° C erwärmt und während einer Stunde bei RT gerührt. Dann wird mit Eiswasser versetzt und zweimal mit je 50 ml Ethylacetat extrahiert. Die vereinigten organ. Extrakte werden mit Wasser gewaschen, über Natriumsulfat getrocknet und das Lösungsmittel unter vermindertem Druck abdestilliert. Der Rückstand wird mit Ethylacetat / Hexan (1:5 Vol% bis 1:4 Vol%) an Kieselgel chromatographisch gereinigt. Man erhält 1,3 g des gewünschten Produktes als farbloses Oel.

### Beispiel H-4:

### Herstellung von 2-(2-(1-(3-(Chloro-difluoro-methoxy)-phenyl)-ethylideneaminoxymethyl)-phenyl)-methoximino-N-methyl-acetamide (I.2)

Man rührt 6,6 g der unter H-3 erhaltenen Verbindung in 15 ml einer 33%igen ethanolischen Methylamin-Lösung während 1 Stunde bei Raumtemperatur. Ethanol und überschüssiges Methylamin werden abdestilliert und der Rückstand mittels Diethylether über Kieselgel filtriert. Man erhält das Produkt in Form eines Oels.

### 2. Formulierungsbeispiele für Wirkstoffe (% = Gewichtsprozent)

| 2.1 Spritzpulver | a) | b) | c) |
|---|---|---|---|
| Wirkstoff | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

| 2.2 Emulsions-Konzentrat | |
|---|---|
| Wirkstoff | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Verdünnung hergestellt werden.

| 2.3 Stäubemittel | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 2.4 Extruder Granulat | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser ange feuchtet. Dieses Gemisch wird extrudiert und anschliessend im Luftstrom getrocknet.

| 2.5 Umhüllungs-Granulat | |
|---|---|
| Wirkstoff | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.6 Suspensions-Konzentrat | |
|---|---|
| Wirkstoff | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl als 75% wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Verdünnung hergestellt werden können.

### 3. Biologische Beispiele:

### Beispiel B-1: Wirkung gegen Puccinia graminis auf Weizen

### a) Residual-protektive Wirkung

Weizenpflanzen werden 6 Tage nach Aussaat mit einer aus Spritzpulver des Wirkstoffes hergestellten wässrigen Spritzbrühe (0,02% Aktivsubstanz) tropfnass besprüht und 24 Stunden später mit einer Uredosporensuspension des Pilzes infiziert. Nach einer Inkubationszeit von 48 Stunden (Bedingungen: 95 bis 100 Prozent relative Luftfeuchtigkeit bei 20°) werden die Pflanzen in einem Gewächshaus bei 22° aufgestellt. 12 Tage nach der Infektion wird der Pilzbefall beurteilt.

### b) Systemische Wirkung

Zu Weizenpflanzen wird 5 Tage nach Aussaat eine_aus Spritzpulver des Wirkstoffes hergestellten wässrigen Spritzbrühe (0,006% Aktivsubstanz, bezogen auf das Bodenvolumen) gegossen. Es wird dabei darauf geachtet, dass die Spritzbrühe nicht mit oberirdischen Pflanzenteilen in Berührung kommt. 48 Stunden später werden die Pflanzen mit einer Uredosporensuspension des Pilzes infiziert. Nach einer Inkubationszeit von 48 Stunden (Bedingungen: 95 bis 100 Prozent relative Luftfeuchtigkeit bei 20°) werden die Pflanzen in einem Gewächshaus bei 22° aufgestellt. 12 Tage nach der Infektion wird der Pilzbefall beurteilt.
Die Verbindungen zeigen eine gute Wirkung.

### Beispiel B-2: Wirkung gegen Phytophthora infestans auf Tomaten

### a) Residual-protektive Wirkung

Tomatenpflanzen werden nach dreiwöchiger Anzucht mit einer aus Spritzpulver des Wirkstoffes hergestellten wässrigen Spritzbrühe (0,02% Aktivsubstanz) tropfnass besprüht und 24 Stunden später mit einer Sporangiensuspension des Pilzes infiziert. Die Beurteilung des Pilzbefalls erfolgt 5 Tage nach der Infektion, während denen 90 bis 100 Prozent relative Luftfeuchtigkeit und eine Temperatur von 20° aufrechterhalten werden.

### b) Systemische Wirkung

Zu Tomatenpflanzen wird nach dreiwöchiger Anzucht eine aus Spritzpulver des Wirkstoffes hergestellten wässrigen Spritzbrühe (0,006% Aktivsubstanz, bezogen auf das Bodenvolumen) gegossen. Es wird dabei darauf geachtet, dass die Spritzbrühe nicht mit oberirdischen Pflanzenteilen in Berührung kommt. Nach 48 Stunden werden die Pflanzen mit einer Sporangiensuspension des Pilzes infiziert. Die Beurteilung des Pilzbefalls erfolgt 5 Tage nach der Infektion, während denen 90 bis 100 Prozent relative Luftfeuchtigkeit und eine Temperatur von 20° aufrechterhalten werden.
Die Verbindungen zeigen eine gute Wirkung.

### Beispiel B-3: Residual-protektive Wirkung gegen Cercospora arachidicola auf Erdnüssen

10 bis 15 cm hohe Erdnusspflanzen werden mit einer aus Spritzpulver des Wirkstoffes hergestellten wässrigen Spritzbrühe (0,02% Aktivsubstanz) tropfnass besprüht und 48 Stunden später mit einer Konidiensuspension des Pilzes infiziert. Die Pflanzen werden 72 Stunden bei 21° und hoher Luftfeuchtigkeit inkubiert und anschliessend bis zum Auftreten der typischen Blattflecken in einem Gewächshaus aufgestellt. Die Bewertung der Wirkung der Aktivsubstanz erfolgt 12 Tage nach Infektion aufgrund von Anzahl und Grösse der Blattflecken.
Die Verbindungen zeigen eine gute Wirkung.

### Beispiel B-4: Wirkung gegen Plasmopara viticola auf Reben

Rebensämlinge im 4 bis 5 Blatt-Stadium werden mit einer aus Spritzpulver des Wirkstoffes hergestellten wässrigen Spritzbrühe (0,02% Aktivsubstanz) tropfnass besprüht und 24 Stunden später mit einer Sporangiensuspension des Pilzes infiziert. Der Pilzbefalls wird 6 Tage nach der Infektion beurteilt, während denen 95 bis 100 Prozent relative Luftfeuchtigkeit und eine Temperatur von 20° aufrechterhalten werden.
Die Verbindungen zeigen eine gute Wirkung.

### Beispiel B-5: Wirkung gegen Colletotrichum lagenarium auf Gurken

Gurkenpflanzen werden nach 2-wöchiger Anzucht mit einer aus Spritzpulver des Wirkstoffs hergestellten Spritzbrühe (Konzentration 0,002%) besprüht. Nach 2 Tagen werden die Pflanzen mit einer Sporensuspension (1.5x10⁵ Sporen/ml) des Pilzes infiziert und für 36 Stunden bei 23°C und hoher Luftfeuchtigkeit inkubiert. Die Inkubation wird dann bei normaler Luftfeuchtigkeit und ca. 22°C weitergeführt. Der eingetretene Pilzbefall wird 8 Tage nach der Infektion beurteilt.
Die Verbindungen zeigen eine gute Wirkung.

### Beispiel B-6: Residual-protektive Wirkung gegen Venturia inaequalis auf Aepfeln

Apfelstecklinge mit 10 bis 20 cm langen Frischtrieben werden mit einer aus Spritzpulver des Wirkstoffes hergestellten wässrigen Spritzbrühe (0,02% Aktivsubstanz) tropfnass besprüht und 24 Stunden später mit einer Konidiensuspension des Pilzes infiziert. Die Pflanzen werden 5 Tage bei 90 bis 100 Prozent relativer Luftfeuchtigkeit inkubiert und weitere 10 Tage in einem Gewächshaus bei 20 bis 24° aufgestellt. 12 Tage nach der Infektion wird der Pilzbefall beurteilt.
Die Verbindungen zeigen eine gute Wirkung.

### Beispiel B-7: Wirkung gegen Erysiphe graminis auf Gerste

### a) Residual-protektive Wirkung

Ungefähr 8 cm hohe Gerstenpflanzen werden mit einer aus Spritzpulver des Wirkstoffes hergestellten wässrigen Spritzbrühe (0,02% Aktivsubstanz) tropfnass besprüht und 3 bis 4 Stunden später mit Konidien des Pilzes bestäubt. Die infizierten Pflanzen werden in einem Gewächshaus bei 22° aufgestellt. 12 Tage nach der Infektion wird der Pilzbefall beurteilt.
Die Verbindungen zeigen eine gute Wirkung.

### b) Systemische Wirkung

Zu ungefähr 8 cm hohen Gerstenpflanzen wird eine aus Spritzpulver des Wirkstoffes hergestellten wässrigen Spritzbrühe (0,002% Aktivsubstanz, bezogen auf das Bodenvolumen) gegossen. Es wird dabei darauf geachtet, dass die Spritzbrühe nicht mit oberirdischen Pflanzenteilen in Berührung kommt. 48 Stunden später werden die Pflanzen mit Konidien des Pilzes bestäubt. Die infizierten Pflanzen werden in einem Gewächshaus bei 22° aufgestellt. 12 Tage nach der Infektion wird der Pilzbefall beurteilt.
Die Verbindungen zeigen eine gute Wirkung.

### Beispiel B-8: Wirkung gegen Podosphaera leucotricha auf Apfeltrieben

Apfelstecklinge mit ca. 15cm langen Frischtrieben werden mit einer Spritzbrühe (0,06% Aktivsubstanz) besprüht. Nach 24Stunden werden die behandelten Pflanzen mit einer Konidiensuspension des Pilzes infiziert und in einer Klimakammer bei 70% relativer Luftfeuchtigkeit und 20°C aufgestellt. 12 Tage nach der Infektion wird der Pilzbefall beurteilt.
Die Verbindungen zeigen eine gute Wirkung.

## Patentansprüche

1. Die Verbindung der Formel I 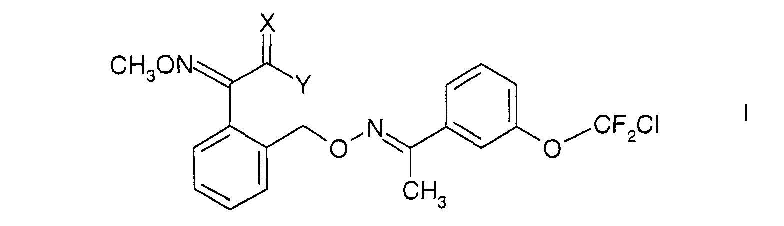 worin bedeuten:
X O und
Y NHCH₃.

2. Die Verbindungen der Formeln IV und III

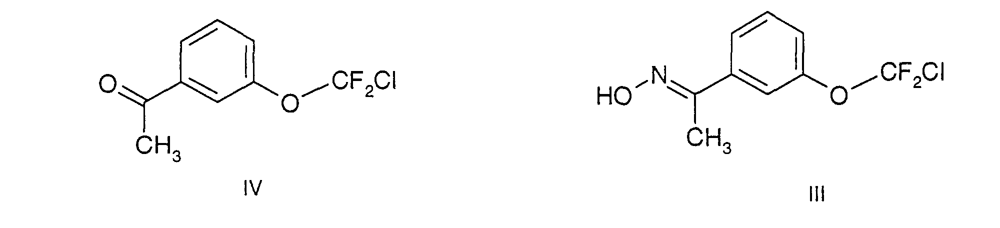

3. Mittel zum Schutz von Pflanzen gegen Pilzbefall, **dadurch gekennzeichnet, dass** es als Wirkstoff die Verbindung gemäss Anspruch 1, in freier Form oder in agrochemisch verwendbarer Salzform, zusamen mit einem Trägermaterial enthält.

4. Verfahren zum Schutz von Pflanzen gegen Pilzbefall, **dadurch gekennzeichnet, dass** man als Wirkstoff die Verbindung gemäss Anspruch 1 auf die Pflanzen, auf Teile der Pflanzen und/oder auf den Standort der Pflanzen appliziert.

## Claims

1. Compound of formula I wherein: 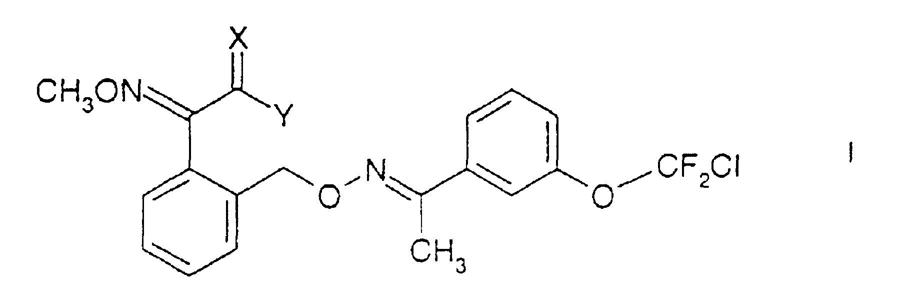
X is O, and
Y is NHCH₃.

2. Compounds of formulae IV and III

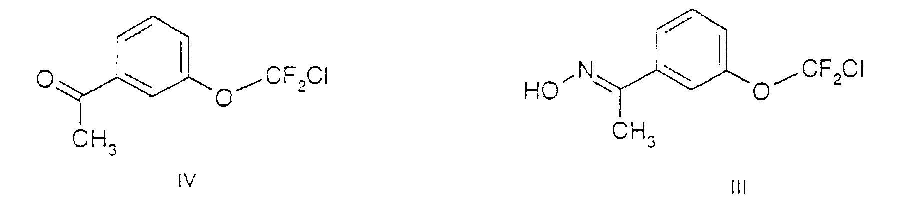

3. Composition for the protection of plants against fungus infestation, **characterized in that** it comprises as active substance the compound according to Claim 1 in free form or in agrochemically usable salt form, together with a carrier.

4. Method of protecting plants against fungus infestation, **characterized in that** as active substance the compound according to Claim 1 is applied to the plants, to parts of the plants and/or to the locus of the plants.

## Revendications

1. Composé de formule I : 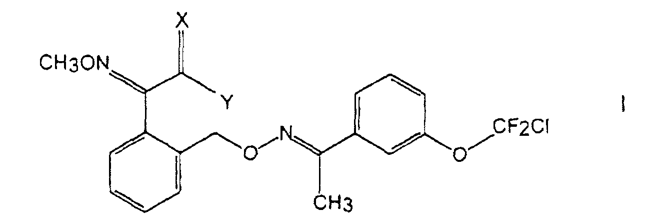 où
X représente l'atome d'O, et
Y représente le radical NHCH₃.

2. Composés des formules IV et III :

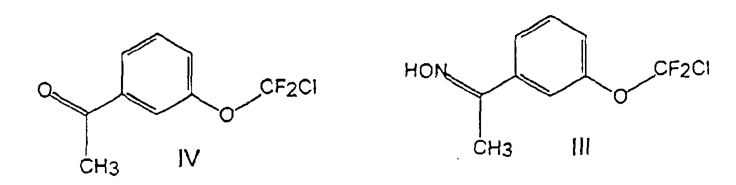

3. Agent de protection des plantes contre une infestation de champignon, **caractérisé en ce qu'**il contient comme agent actif, le composé suivant la revendication 1, sous forme libre ou sous une forme saline utilisable en agrochimie, avec un matériau support.

4. Procédé de protection des plantes contre une infestation de champignon, **caractérisé en ce que** l'on applique comme agent actif, le composé suivant la revendication 1, sur les plantes, sur des parties des plantes et/ou sur le lieu d'implantation des plantes.
